Europäisches Patentamt

European Patent Office

Office européen des brevets

(11) Publication number: **0 256 146**
**A1**

# EUROPEAN PATENT APPLICATION

(21) Application number: 86111102.9

(22) Date of filing: 11.08.86

(51) Int. Cl.4: **C07D 213/61**

(43) Date of publication of application:
24.02.88 Bulletin 88/08

(84) Designated Contracting States:
BE CH DE FR GB IT LI NL

(71) Applicant: THE DOW CHEMICAL COMPANY
2030 Dow Center Abbott Road P.O. Box 1967
Midland, MI 48640(US)

(72) Inventor: Fung, Alexander P.
1941 Spring Lake Drive
Martinez California 94553(US)

(74) Representative: Weickmann, Heinrich,
Dipl.-Ing. et al
Patentanwälte Dipl.-Ing. H.Weickmann
Dipl.-Phys.Dr. K.Fincke Dipl.-Ing.
F.A.Weickmann Dipl.-Chem. B. Huber Dr.-Ing.
H. Liska Dipl.-Phys.Dr. J. Prechtel Postfach
860820
D-8000 München 86(DE)

(54) Catalyst recycle in liquid phase halogen exchange reaction.

(57) (Trifluoromethyl)pyridines prepared by the metal halide catalyzed fluorine for chlorine exchange in the corresponding trichloromethylpyridines are directly separated from the reaction vessel by distillation and the residue from the distillation is employed to catalyze additional fluorine for chlorine exchange.

## CATALYST RECYCLE IN LIQUID PHASE HALOGEN EXCHANGE REACTION

The preparation of (trifluoromethyl)pyridine compounds by reacting a trichloromethylpyridine compound with gaseous hydrogen fluoride is described in U.S. Patent 4,184,041.

U.S. Patent 4,590,279 provides an improvement in that process by requiring that the liquid phase reaction is carried out in the presence of a metal halide or metal halide/phosphorus halide catalyst. While this process provides good yields of the desired products, undesirable amounts of organic fluoride waste are produced.

In accordance with this invention the amount of organic fluoride waste produced in the metal halide catalyzed preparation of (trifluoromethyl)pyridine compounds from trichloromethylpyridine compounds is reduced and the isolated yields of the desired (trifluoromethyl)pyridine compounds are increased by directly separating the desired (trifluoromethyl)pyridine products from the reaction mass by distillation and by employing the residue of said distillation to catalyze the fluorine for chlorine exchange in fresh starting material.

In the practice of the present invention a (trichloromethyl)pyridine compound is contacted with hydrogen fluoride and a catalytic amount of a metal halide or a metal halide/phosphorus halide catalyst, hereinafter referred to as "catalyst", under liquid phase reaction conditions, the product (trifluoromethyl)pyridine compound is recovered by distillation, either flash distillation or through a short rectifying column, and the residue of said distillation is used to catalyze further fluorine for chlorine exchange.

(Trichloromethyl)pyridine compounds employed as the starting material as unsubstituted or substituted-(trichloromethyl)pyridine compounds containing one or two trichloromethyl groups. The trichloromethyl groups may be in $\alpha$, $\beta$ or $\gamma$ ring positions. The pyridine ring optionally contains other substituents, besides the $CCl_3$ groups, which do not affect the halogen exchange reaction of this invention. Such substituents include, for example, Cl, Br, I or F. Preferred (trichloromethyl)pyridine compounds include mono-or dichloro-$\beta$-trichloromethyl pyridine, such as, 2,3-dichloro-5-(trichloromethyl)pyridine; 2-chloro--5-(trichloromethyl)-pyridine; and 2,6-dichloro-3-(trichloromethyl)pyridine.

Hydrogen fluoride is employed as the source of fluorine in the present reaction. The hydrogen fluoride is introduced into the present reaction as hydrogen fluoride (anhydrous) or as hydrofluoric acid. The hydrogen fluoride is bubbled into the reaction as a gas or fed into the reaction as a liquid. Hydrogen fluoride (anhydrous) has a boiling point of 19.5°C and the liquid and gas consist of associated molecules. Hydrogen fluoride (anhydrous) is a well-known compound and commercially available, generally in cylinders and tank cars. Hydrogen fluoride is also supplied as hydrofluoric acid which is hydrogen fluoride in aqueous solution. In the practice of the present invention, hydrogen fluoride is contacted with the other reactants and preferably hydrogen fluoride (anhydrous) is employed as the hydrogen fluoride source. Hydrogen fluoride is supplied at a ratio of at least 3 moles per mole of mono-(trichloromethyl)pyridine compound and preferably an excess of this amount is employed. When bis-(trichloromethyl)pyridine compounds are employed as starting materials at least 6 moles of HF per mole of bis-(trichloromethyl)pyridine compound are required to fluorinate the 2 trichloromethyl groups while it is preferred to supply an excess of this amount.

Metal halides are employed in catalytic amounts in the present reaction. Suitable metal halides include metal chlorides and metal fluorides. Suitable metal chlorides include $FeCl_2$, $FeCl_3$, $NbCl_5$, $TaCl_5$ or mixtures thereof. Suitable metal fluorides include $SbF_3$, $FeF_2$, $FeF_3$, $SnF_4$, $TiF_4$, $CrF_2$ or mixtures thereof. The metal halide catalysts are added to the present reaction in catalytic amounts, generally from 0.1 to 20 mole percent based on the amount of (trichloromethyl)pyridine compound starting material present, and preferably from 0.5 to 10 mole percent. Preferred metal halide catalysts include $FeCl_3$, $FeF_3$, $FeCl_2$, and $FeF_2$. Especially preferred metal halide catalysts are $FeCl_3$, $FeF_3$ and mixtures thereof.

Also, acceptable as a catalyst is a metal halide/phosphorus halide combination. Such a combination is achieved by supplying a phosphorus halide to the reaction mixture in addition to the metal halide catalyst. A preferred phosphorus halide is $PCl_5$.

Catalysts bonded to inert supports or precursor compounds which form the catalysts in situ are contemplated for use in the present invention. Examples of inert supports to which the catalysts may be bonded include graphite, alumina, various clays and molecular sieves which are all well known in the art.

The present reaction is conducted under liquid phase conditions at a temperature usually under 250°C, preferably at a temperature between 100°C and 250°C. It is especially preferred to conduct the present reaction at a temperature between 170°C and 180°C. The present halogen exchange reaction is typically conducted in the presence of agitation sufficient to maintain an essentially homogenous mixture of the reactants. The pressure at which the present reaction is carried out is not critical, as it will proceed at a variety of pressures from atmospheric to superatomospheric pressure. It is convenient to conduct the reaction at ambient atmospheric pressure.

In conducting the present reaction the order of addition of the reactants is not critical. Preferably, the (trichloromethyl)pyridine compound and the catalyst are admixed to form a reaction mixture and thereafter the hydrogen fluoride is added into this mixture, with stirring, until the reaction is completed, generally in from about 1 to about 100 hours. The exact time that the reaction is complete will vary on a variety of factors, such as, temperature, catalyst concentration, HF flow rate, degree of agitation and pressure. The hydrogen fluoride is fed into the reaction mixture as a liquid or, alternatively, may be bubbled or sparged into the reaction mixture as a gas.

In a preferred operation, 2,3-dichloro-5-(trichloromethyl)pyridine is mixed with a catalytic amount of $FeCl_3$ or $FeCl_2$ and then hydrogen fluoride (anhydrous) is continuously added into the reaction mixture while the temperature is increased to from 150°C to 190°C and preferably from 170°C to 180°C. The reaction is usually completed in from about 1 to about 48 hours. The hydrogen fluoride (anhydrous) and HCl which escapes from the reaction mixture as vapor is conveniently collected employing conventional techniques such as by condensation. Thus the HF may be liquefied and returned to the reactor allowing the more volatile HCl to escape, thus conserving the amount of HF used and driving the reaction to completion.

A unique aspect of the present invention is presented when the desired (trifluoromethyl)pyridine compound which is being prepared has a boiling point below the temperature at which the reaction is conducted. When this occurs, the (trifluoromethyl)pyridine product vaporizes as it is formed and is conveniently collected in a pure form, separate from any (trichloromethyl)pyridine starting materials which generally have boiling points greater than the temperature at which the reaction is conducted. This allows the reaction to be run continuously by the substantially continuous addition or feeding of (trichloromethyl)-pyridine compound and hydrogen fluoride to the reaction mixture.

In a further aspect of the present invention, any ring fluorinated materials may be reconverted to ring chlorinated products by treatment with a chlorinating agent as described in U.S. Patent 4,546,192 without adding further catalyst.

The invention is further illustrated by the following examples.

## Example 1

A 480 ml Telfon PFA reactor flask containing a magnetic stir bar and fitted with a thermocouple, an HF/HCl bleed tube and a PFA reflux condenser which was connected through a PFA cold trap to a caustic scrubber was charged with 180 grams of 2,3-dichloro-5-trichloromethylpyridine and 5.5 grams (5 mole %) of $FeCl_3$. Anhydrous HF gas was continuously introduced into the reaction mixture which was heated to 175°C and maintained at 170°C-175°C for 55 hours. At that time the mixture analyzed 61.2 weight percent (GLC) 2,3-dichloro--5-(trifluoromethyl)pyridine and 20.1 weight percent 3-chloro-2-fluoro-5-(trifluoromethyl)-pyridine with minor amounts of other products.

The reaction temperature was lowered to 140°C and kept there for an additional 15 hours while continuously sparging in anhydrous HCl at a rate of 25 cc/minute. No fresh catalyst was added. Analysis showed 79.4 weight percent 2,3-dichloro-5-(trifluoromethyl)pyridine and only 1.8 weight percent 3-chloro--2-fluoro-5-(trifluoromethyl)pyridine.

The product mixture (134 grams) was cooled down to room temperature and transferred into a 250 ml distillation flask for distillation under vacuum (glassware - 4 tray). The 111 grams of overheads were collected at 70°-100°C at 135-140 mm Hg. Greater than 98 percent of the available trifluoromethyl product was recovered.

3

Example 2

The reaction flask employed in Example 1 was charged with 175 grams of a mixture containing:

|  | Wt % |
| --- | --- |
| 3-chloro-2-fluoro-5-(trifluoromethyl)pyridine - | 32.5 |
| 2,3-dichloro-5-(trifluoromethyl)pyridine - | 48.7 |
| 3-chloro-2-fluoro-5-chlorodifluoromethylpyridine - | 6.0 |
| 2,3-dichloro-5-chlorodifluoromethylpyridine - | 8.3 |

and about 5 mole percent of FeCl₃. The mixture was heated to 140°C and anhydrous HCl was continuously introduced at a rate of 25 cc/minute for 32 hours. After cooling to room temperature the mixture (172 grams) was transferred into a 250 ml distillation flask and distillation (glassware - 4 tray) was continued until the pot temperature reached 160°C at 100 mm Hg. The overheads (150.1 grams) were collected at 65° to 105°C at 100-105 mm Hg. The residue (20.2 grams) was combined with 171.8 grams of fresh 2,3-dichloro-5-trichloromethylpyridine and a second cycle carried out employing the procedure described in Example 1. The residue from the distillation from this run (17 grams) was combined with 163 grams of 2,3-dichloro-5-(trichloromethyl)pyridine and the whole cycle was repeated again. The chemical yields from the second and third cycles were 87.8 percent and 87.0 percent respectively. The isolated yields in both cases were greater than 85 percent.

## Claims

1. Process for preparing a mono-or dichloro-(trifluoromethyl)pyridine compound by contacting a mono- or dichloro-(trichloromethyl)pyridine compound with hydrogen fluoride in the presence of a catalytic amount of a metal halide catalyst in a liquid phase reaction, characterized by recovering the (trifluoromethyl)pyridine compound by distillation and using the distillation residue to catalyze further fluorine for chlorine exchange.

2. Process of Claim 1 characterized in that the reaction is carried out at a temperature under 250°C.

3. Process of Claim 2 characterized in that the hydrogen fluoride is anhydrous.

4. Process of Claim 3 characterized in that the catalyst is FeCl₃ which is present in the reaction mixture in an amount of from 0.1 to 20 mole percent based on the molar quantity of (trichloromethyl)pyridine present in the reaction mixture.

5. Process of Claim 4 characterized in that the (trifluoromethyl)pyridine compound is 2,3-dichloro-5-(trifluoromethyl)pyridine and the (trichloromethyl)pyridine compound is 2,3-dichloro--5-(trichloromethyl)-pyridine.

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 184 041 (R. NISHIYAMA et al.)<br>* Column 4, lines 11-26 *<br>--- | 1 | C 07 D 213/61 |
| Y | EP-A-0 110 690 (DOW)<br>* Page 22, lines 17-25; page 32, claims 1,2 * & US-A-4 590 279 (Cat. D)<br>--- | 1 | |
| Y | KIRK-OTHMER: "Concise encyclopedia of chemical technology", 1985, page 224, John Wiley & Sons, New York, US<br>* Page 224, column 2, lines 28-39 *<br>----- | 1 | |

**TECHNICAL FIELDS SEARCHED (Int. Cl.4)**

C 07 D 213/00

The present search report has been drawn up for all claims

| Place of search | Date of completion of the search | Examiner |
|---|---|---|
| THE HAGUE | 13-04-1987 | BRENNAN J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82